# EUROPEAN PATENT APPLICATION

(11) **EP 4 252 748 A1**
(43) Date of publication of application: **04.10.2023**
(21) Application number: 22166162.2
(22) Date of filing: 31.03.2022
(51) Int. Cl.: A61K 31/137, A61K 31/397, A61K 31/4245, A61K 45/06, A61P 25/28

(54) **A SPHINGOSINE-1-PHOSPHATE RECEPTOR (S1PR) MODULATOR FOR USE IN TREATING A PATIENT SUFFERING FROM ALZHEIMER'S DEMENTIA**

(71) Applicant: Otto-von-Guericke-University Magdeburg, 39106 Magdeburg (DE)
(72) Inventor: The designation of the inventor has not yet been filed

(57) **Abstract**

The present invention relates to a sphingosine-1-phosphate receptor (S1PR) modulator for use in treating a patient suffering from Alzheimer's dementia.

## Description

The present invention relates to a sphingosine-1-phosphate receptor (S1PR) modulator for use in treating a patient suffering from Alzheimer's dementia.

### BACKGROUND

Dementia is one of the greatest global challenges for health and social care in the 21st century. It occurs mainly in people older than 65 years. Globally, about 50 million people are currently living with dementia, and this number is projected to triple by 2050. The 2015 global cost of dementia was >800 billion € per year, and this figure will continue to increase as the number of people with dementia rises.

Alzheimer's disease (AD) is the most common (~60%) form of dementia characterized by a progressive decline of cognitive functions. Pathologically, AD is characterized by amyloid plaques and neurofibrillary tangles in the brain, with associated loss of synapses and neurons, resulting in cognitive deficits and eventually dementia. While lifestyle factors such as physical exercise or a healthy diet might be able to delay the disease onset, >30 years of dementia research have only very recently led to a single drug (aducanumab, FDA approved 2021) for causal treatment of dementia in AD patients.

Increasing evidence suggests that AD pathogenesis is not restricted to neurons, but includes strong interactions with immunological mechanisms in the brain. Misfolded and aggregated proteins bind to pattern recognition receptors on microglia and astroglia, and trigger an innate immune response characterized by the release of inflammatory mediators, which contribute to disease progression and severity. This inflammatory response is also present in the ~40% dementia patients that cannot be assigned unequivocally as AD patients, but which show similar synaptic dysfunctions, and would therefore also benefit from treatments which tackle neuroinflammation. Accordingly, therapeutic approaches focusing on reduction in neuroinflammatory signaling are among the most promising interventions to ameliorate AD-related deficits, and deficits observed in other types of dementia (e.g., vascular dementia, mixed AD and vascular dementia, frontotemporal dementia, dementia with Lewy bodies). Therefore, repurposing FDA-approved anti-inflammatory drugs for therapy of AD and other types of dementia could provide an important benefit to rapidly develop an effective AD/dementia medication.

Fingolimod (FTY720, Gilenya^{®}) is an approved (FDA and EMA) drug for the treatment of relapsing multiple sclerosis (RMS) since 2011. In target tissue, it can be converted to fingolimod-phosphate, which is a potent modulator of sphingosine-1-phosphate receptors (S1PR1-5). The sphingosine-1-phosphate receptors are a family of seven helix transmembrane G protein-coupled receptors that recognize and bind extracellular sphingosine-1-phosphate to affect cellular processes. They are divided into five subtypes: S1PR1, S1PR2, S1PR3, S1PR4, and S1PR5. In the immune system, fingolimod-induced S1PR receptor modulation promotes the retention of T-cells in lymph nodes, thereby reducing the invasion of the central nervous system by auto-reactive lymphocytes, where they attack the protective sheath (myelin) that covers nerve fibers and cause permanent damage or deterioration of the nerves.

At present, there is no treatment for Alzheimer's dementia known. In particular, there is no treatment for Alzheimer's dementia known that can stop AD progression if the disease is not in its onset but already in a late stage.

The present invention is based on the surprising finding that treatment with modulators of sphingosine-1-phosphate receptors (such as fingolimod, FTY720) may stop progression of synaptic deficits and memory dysfunction in a late state of AD. Even more surprising is the finding, that treatment with S1PR modulators is even effective to revert AD. The S1PR modulators can also be used in combination therapy.

### SUMMARY OF THE INVENTION

In a first aspect, the present relates to a sphingosine-1-phosphate receptor (S1PR) modulator for use in treating a patient suffering from Alzheimer's dementia. The S1PR modulator may be selected from the group consisting of fingolimod (FTY720), siponimod (BAF312), ozanimod (RPC1063), ceralifimod (ONO-4641), GSK2018682, ponesimod (ACT128800), and amiselimod (MT-1303).

In a second aspect, the invention relates to a combination for use in treating a patient suffering from Alzheimer's dementia, wherein the combination comprises
(i) an S1PR modulator, and
(ii) another therapeutic substance.
The other therapeutic substance may be selected from the group consisting of aducanumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

In a third aspect, the present invention relates to a pharmaceutical composition comprising the sphingosine-1-phosphate receptor (S1PR) modulator as defined in the first aspect or the combination as defined in the second aspect for use in treating a patient suffering from Alzheimer's dementia.

In a fourth aspect, the present invention relates to a method for treating Alzheimer's dementia comprising the steps of
(i) providing an S1PR modulator or a combination comprising an S1PR modulator and another therapeutic substance, and
(ii) administering a patient suffering from Alzheimer's dementia the S1PR modulator or the combination comprising an S1PR modulator and another therapeutic substance in a therapeutically effective amount.

This summary of the invention does not necessarily describe all features and/or all aspects of the present invention. Other embodiments will become apparent from a review of the ensuing detailed description.

### DETAILED DESCRIPTION

### Definitions

In the following the invention is described in more detail with reference to the Figures. The described specific embodiments of the invention, examples, or results are, however, intended for illustration only and should not be construed to limit the scope of the invention as indicated by the appended claims in any way.

It is to be understood that this invention is not limited to the particular methodology, protocols, and reagents described herein as these may vary. It is also to be understood that the terminology used herein is to describe particular embodiments only, and is not intended to limit the scope of the present invention which will be limited only by the appended claims. Unless defined otherwise, all technical and scientific terms used herein have the same meanings as commonly understood by one of ordinary skill in the art.

Each of the documents cited in this specification (including all patents, patent applications, scientific publications, manufacturer's specifications, instructions, etc.), whether supra or infra, is hereby incorporated by reference in its entirety. In the event of a conflict between the definitions or teachings of such incorporated references and definitions or teachings recited in the present specification, the text of the present specification takes precedence.

The term "comprising" or variations thereof such as "comprise(s)" according to the present invention (especially in the context of the claims) is to be construed as an open-ended term or non-exclusive inclusion, respectively (i.e., meaning "including, but not limited to,") unless otherwise noted.

The terms "a", "an", and "the" as used herein in the context of describing the invention (especially in the context of the claims) should be read and understood to include at least one element or component, respectively, and are to be construed to cover both the singular and the plural, unless otherwise indicated herein or clearly contradicted by context.

In addition, unless expressly stated to the contrary, the term "or" refers to an inclusive "or" and not to an exclusive "or" (i.e., meaning "and/or").

All numeric values are herein assumed to be modified by the term "about", whether or not explicitly indicated. Recitation of ranges of values herein is merely intended to serve as a shorthand method of referring individually to each separate value falling within the range, unless otherwise indicated herein, and each separate value is incorporated into the specification as if it were individually recited herein.

The use of terms "for example", "e.g.", "such as", or variations thereof is intended merely to better illuminate the invention and does not pose a limitation on the scope of the invention unless otherwise claimed. These terms should be interpreted to mean "but not limited to" or "without limitation".

The term "dementia", as used herein, is a general term for a decline in mental ability severe enough to interfere with daily life. Dementia describes a group of symptoms associated with a decline in memory, reasoning or other thinking skills. Many different types of dementia exist, and many conditions cause it. Mixed dementia is a condition in which brain changes of more than one type of dementia occur simultaneously. Alzheimer's disease is the most common cause of dementia, accounting for 60-80% (i.e. AD, or mixed AD + vascular dementia) of dementia cases. Alzheimer's is a specific disease. Dementia is not.

The term "Alzheimer's disease (AD)", as used herein, refers to a neurodegenerative disease. It is the most common form of dementia. Alzheimer's disease is characterized by loss of neurons and synapses in the cerebral cortex and certain subcortical regions and leads to a gross degeneration in these regions. In Alzheimer's disease, protein misfolding and aggregation (formation of so-called "plaques") in the brain is caused by accumulation of abnormally folded A-beta and tau proteins in the affected tissues. The most common early symptom is difficulty in remembering recent events (short-term memory loss). As the disease advances, symptoms can include problems with language, disorientation, mood swings, loss of motivation, not managing self-care and behavioral issues. As a person's condition declines, they often withdraw from family and society. Gradually, bodily functions are lost, ultimately leading to death.

The term "Alzheimer's dementia", as used herein, refers to a specific form of Alzheimer's disease.

Alzheimer's disease (AD), the major cause of dementia among the elderly world-wide, manifests in familial and sporadic forms, and the latter variety accounts for the majority of the patients affected by this disease.

The term "sporadic Alzheimer's dementia", as used herein, refers to a multi-factorial disorder in which both genetic predisposition and environmental factors contribute to the genesis of the disease. The majority (more than 95 %) of the AD dementia cases belong to sporadic variety of the disease. Sporadic Alzheimer's disease can affect adults at any age, but usually occurs after age 65.

The term "familial Alzheimer's dementia", as used herein, refers to a disease which arises from the mutations of any of the three genes e.g. amyloid precursor protein *(APP),* presenilin 1 *(PS* 1) and presenilin 2 *(PS 2)* and usually appears in somewhat younger age-group than the sporadic form and follows a more aggressive downhill course. The minority (less than 5%) of the AD dementia cases belong to familial Alzheimer's dementia.

The term "Sphingosine-1-phosphate (SIP)", as used herein, refers to a signaling molecule involved in, among other things, lymphocyte migration, brain and heart development, vascular permeability, and regulation of vascular and bronchial tone. In this regard, SIP acts through a series of membrane-bound SIP receptors, in particular SIP receptor subtypes (S1PR1, 2, 3, 4, 5) that exhibit variable organ- and cell-specific expression patterns and thus have distinct functions.

The term "Sphingosine-1-phosphate receptor (S1PR)", as used herein, refers to a receptor bound by S1P.

In biochemistry and pharmacology, a "receptor modulator" is a general term for a substance, endogenous or exogenous, that binds to and regulates the activity of certain cell receptors, i.e. protein structures, that receive and transduce signals and cause some form of cellular response. Receptor modulators can act on different parts of receptors and regulate activity in a positive, negative, or neutral direction with varying degrees of efficacy. Categories of these receptor modulators include receptor agonists and receptor antagonists. Generally, an agonist is a modulator (e.g. drug) that binds to a receptor and produces a similar response to the intended (natural) binding ligand. An antagonist is a modulator (e.g. drug) that binds to the receptor and stops the receptor from producing a cellular response.

The term "Sphingosine-1-phosphate receptor (S1PR) modulator", as used herein, refers to molecules that bind to SIP receptors, e.g. on lymphocytes, and lead to internalization of the receptor. It is thought that there is a subsequent loss of sensitivity to the SIP gradient, which causes the lymphocytes to remain in the lymph nodes. Their circulating numbers decrease and inflammatory cell migration to the central nervous system decreases. In addition, direct effects of S1PR modulators on the blood-brain barrier, microglia, astrocytes, and oligodendrocytes may be relevant.

The S1PR modulator preferably used in the present invention is selected from the group consisting of fingolimod (FTY720), siponimod (BAF312), ozanimod (RPC1063), ceralifimod (ONO-4641), GSK2018682, ponesimod (ACT128800), and amiselimod (MT-1303).

The term "patient", as used herein, refers to any individual which may receive the S1PR modulator or the combination comprising an S1PR modulator and another therapeutic substance, as described herein. In particular, the term "subject", as used herein, refers to any individual that/who may benefit from the treatment with the the S1PR modulator or the combination comprising an S1PR modulator and another therapeutic substance, as described herein. Specifically, the term "patient" refers to a subject that is or will be receiving, or has received, medical treatment for a disease or condition, i.e. Alzheimer's dementia in the context of the present invention. Preferably, the Alzheimer's dementia is sporadic Alzheimer's dementia or familial Alzheimer's dementia.

The patient may be a vertebrate, e.g. a human being, dog, cat, sheep, goat, cow, horse, camel or pig. It is particularly preferred that the "patient" is a human being.

The term "treatment", in particular "therapeutic treatment", as used herein, refers to any therapy which improves the health status and/or prolongs (increases) the lifespan of a subject suffering from a disease. Said therapy may eliminate the disease in a subject, arrest or slow the development of the disease in a subject, inhibit the development of the disease in a subject, decrease the severity of symptoms in a subject suffering the disease, and/or decrease the recurrence in a subject who currently has or who previously has had a disease.

As used herein, the expressions "is for administration" and "is to be administered" have the same meaning as "is prepared to be administered". In other words, the statement that an active compound "is for administration" has to be understood in that said active compound has been formulated and made up into doses so that said active compound is in a state capable of exerting its therapeutic activity. In the context of the present invention, the S1PR modulator or a composition comprising the S1PR modulator, as described herein, can be prepared for administration.

In the present invention, S1PR modulators, or compositions comprising them, are generally administered in a "therapeutically (pharmaceutically) effective" amount. The term "therapeutically (pharmaceutically) effective" amount, as used herein, generally refers to the amount of a drug that achieves the desired reaction or the desired effect alone or together with further doses. In the case of the treatment of a particular disease, the desired reaction preferably relates to an inhibition of the course of the disease. This comprises slowing down the progress of the disease and, in particular, interrupting or reversing the progress of the disease. The desired reaction in treatment of a disease may also be a delay of the onset or prevention of the onset of the disease. A "therapeutically (pharmaceutically) effective" amount of a drug will generally depend on the condition of the patient to be treated, the severeness of the disease, the individual parameters of the patient, including age, physiological condition, size, and weight, the duration of treatment, the type of an accompanying therapy (if present), the specific route of administration, and similar factors. In case reaction of the patient is insufficient with an initial dose, higher doses (or effectively higher doses achieved by a different, more localized route of administration) may be used.

The pharmaceutical composition as described herein preferably further comprises one or more excipient(s),

The term "excipient", as used herein, is intended to indicate all substances in a pharmaceutical composition which are not active ingredients such as binders, lubricants, thickeners, surface active agents, preservatives, emulsifiers, buffers, flavoring agents, or colorants.

The term "diluent", as used herein, relates to a diluting and/or thinning agent. Moreover, the term "diluent" includes a solution, suspension (e.g. liquid or solid suspension) and/or media.

The term "carrier", as used herein, relates to one or more compatible solid or liquid fillers, which are suitable for an administration, e.g. to a human. The term "carrier" relates to a natural or synthetic organic or inorganic component which is combined with an active component in order to facilitate the application of the active component, or the permeation of the active component to the intended site of action. Preferably, carrier components are sterile liquids such as water or oils, including those which are derived from mineral oil, animals, or plants, such as peanut oil, soy bean oil, sesame oil, sunflower oil, etc. Salt solutions and aqueous dextrose and glycerin solutions may also be used as aqueous carrier compounds. A carrier compound that improves or facilitates permeation of the skin in topical applications is dimethyl sulfoxide (DMSO). Another preferred carrier consists of layered double hydroxide (LDH) nanoparticles. For example, such an LDH nanoparticles can be of the form [Mg3Al(OH)8](CH3CHOHCOO), that can be obtained, for example, by a reaction of Mg(lactate)₂ 3H2O and Al(lactate)₃ in NaOH-controlled pH at 45-65 °C and protected from CO₂ and carbonic acids. A circularized RNA may intercalate into LDH nanoparticles and can be adminstered as LDH nanoparticle or be co-administered with VLPs.

Pharmaceutical carriers, diluents, and/or excipients can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, lactose, sucrose, trehalose, corn sweeteners, natural and synthetic gums, such as acacia, tragacanth or sodium alginate, carboxymethyl cellulose, and polyethylene glycol. Examples of suitable lubricants include sodium oleate, sodium stearate, magnesium stearate, sodium benzoate, sodium acetate, sodium chloride, and the like. Preservatives, stabilizers, dyes, and even flavoring agents may be provided in the pharmaceutical composition. Examples of preservatives include sodium benzoate, sorbic acid, and esters of p-hydroxybenzoic acid. Antioxidants and suspending agents may be also used.

No language in this specification should be construed as indicating any non claimed element as essential to the practice of the invention.

### Embodiments

The present invention is based on the surprising finding that treatment with modulators of sphingosine-1-phosphate receptors (such as fingolimod, FTY720) may stop progression of synaptic deficits and memory dysfunction in a late state of AD. Even more surprising is the finding, that treatment with S1PR modulators is even effective to revert AD. The S1PR modulators can also be used in combination therapy.

Thus, in a first aspect, the invention relates to a sphingosine-1-phosphate receptor (S1PR) modulator for use in treating a patient suffering from Alzheimer's dementia.

The S1PR modulators used in the invention may be selected from the group consisting of fingolimod (FTY720), siponimod (BAF312), ozanimod (RPC1063), ceralifimod (ONO-4641), GSK2018682, ponesimod (ACT128800), and amiselimod (MT-1303). Fingolimod (FTY720) is a preferred S1PR modulator.

Fingolimod (FTY720, tradename Gilenya^{®}) has the formula:

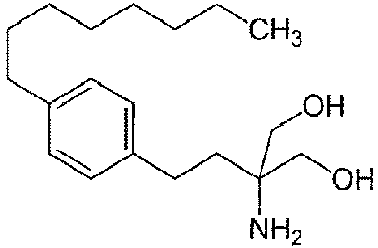

Siponimod (BAF312) has the formula:

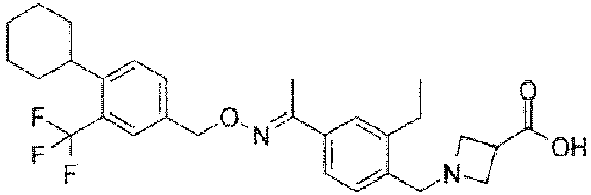

Ozanimod (RPC1063, tradename Zeposia^{®}) has the formula:

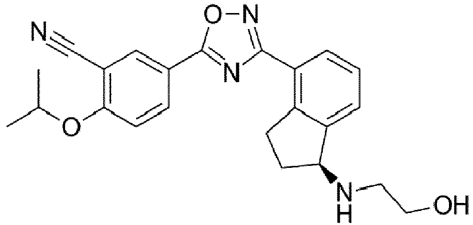

Ceralifimod (ONO-4641) has the formula:

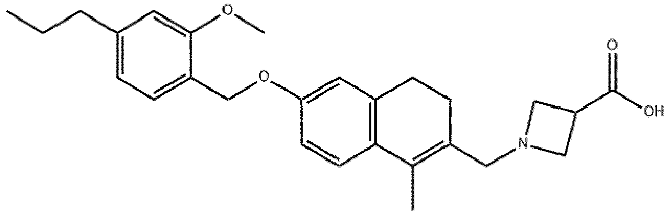

GSK2018682 has the formula:

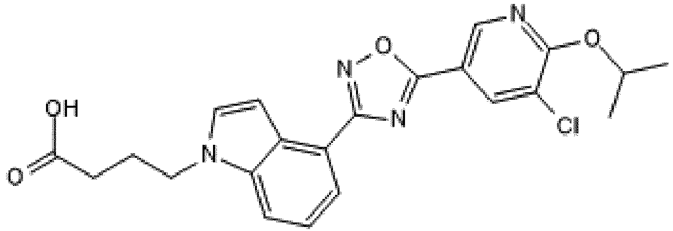

Ponesimod (ACT128800) has the formula:

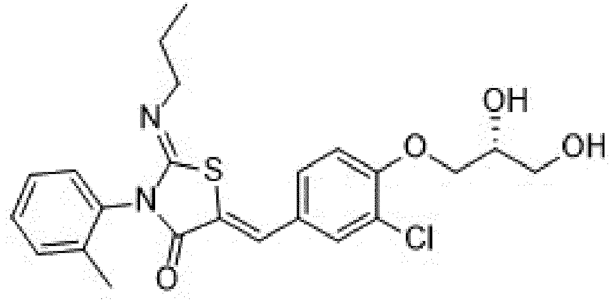

Amiselimod (MT-1303) has the formula:

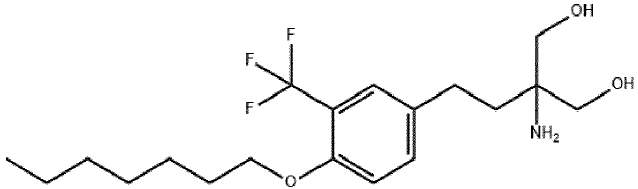

The above S1PR modulators are so far used as medicaments to treat multiple sclerosis or inflammatory bowel disease.

The skilled person appreciates that certain of the above S1PR modulators may have different optical isomers as a result of one or more asymmetric or chiral carbon atoms. Further, some S1PR modulators may display polymorphism. It is also clear that pharmaceutically acceptable salts, esters, solvates, or hydrates of the above S1PR modulators as well as prodrugs that may convert *in vivo* into the above S1PR modulators may be used in the invention.

Examples for pharmacologically acceptable salts of the above S1PR modulators are salts of sufficiently basic S1PR modulators with physiologically acceptable mineral acids like hydrochloric, sulfuric or phosphoric acid; or salts of organic acids like acetic, lactic, citric, succinic, fumaric, maleic or salicylic acid. Further, sufficiently acid S1PR modulators may form alkali or earth alkaline metal salts, for example, sodium, potassium, lithium, calcium or magnesium salts; ammonium salts; or salts of an organic base, for example, methylamine, dimethylamine, trimethylamine, triethylamine, ethylenediamine, ethanolamine, choline hydroxide, piperidine, morpholine, tris-(2-hydroxyethyl)amine, lysine or arginine salts.

Pharmacologically acceptable esters of the above S1PR modulators are esters that can be hydrolyzed by esterase enzyms in the human body into a S1PR modulator with the above formula.

Pharmacologically acceptable solvates of the above S1PR modulators are complex compounds (adducts) formed by the interaction of the molecules of a solvent with molecules of the above S1PR modulators. Hydrates are solvates where the solvent is water.

According to the invention, a sphingosine-1-phosphate receptor (S1PR) modulator (such as fingolimod) may be used in treating a patient suffering from Alzheimer's dementia, wherein said patient is about 40 years old or older.

Alzheimer's dementia to be treated may be sporadic Alzheimer's dementia or familial Alzheimer's dementia. Familial Alzheimer's disease is a rare special form (1-2%) of classic Alzheimer's disease with an earlier onset of the disease and may start at an age of about 40 years, while sporadic Alzheimer's disease starts usually at an age of 60 or older.

Thus, according to the invention, a sphingosine-1-phosphate receptor (S1PR) modulator (such as fingolimod) may be used in treating a patient suffering from sporadic Alzheimer's dementia, wherein the patient may be about 60 years or older, or, in case of a patient suffering from familial Alzheimer's dementia, the patient may be about 40 years or older.

The skilled person appreciates that the above ages of the Alzheimer patients are only very approximate, as the boundaries between the outbreak of the diseases can overlap.

In one embodiment of the invention, the patient to be administered a sphingosine-1-phosphate receptor (S1PR) modulator (such as fingolimod) is a patient who has received or is receiving behavioral therapy, physical exercise training, reality orientation training, physiotherapy, occupational therapy, or cognitive training (memory training), or a combination thereof.

According to the invention, an S1PR modulator (such as fingolimod) may be used to reverse Alzheimer's dementia. This is a very surprising finding of the invention.

In the invention, the administration route of an S1PR modulator is not subject to any particular restrictions and may be enteral or parenteral. Examples are intraperitoneal or oral administration.

The skilled person appreciates that S1PR modulators may be administered as such or in the form of a pharmaceutical composition or formulation comprising an S1PR modulator as the active agent and, optionally, pharmaceutically acceptable excipients commonly used in galenics (e.g., carriers, fillers, diluents, absorbents, binders, adjuvants, etc).

For oral administration, the S1PR modulators may be formulated, for example, as tablets (including effervescent tablets, sustained-release tablets, controlled-release tablets, or entericcoated tablets), dragees, pills, granules, powders, gels, semisolids, capsules (including soft and hard capsules, sustained-release capsules, controlled-release capsules or enteric coated capsules). The above oral administration forms may be prepared following standard galenic procedures, for example, using a pharmaceutically acceptable carrier well known in the art. The pharmaceutically acceptable carriers may include fillers, absorbents, wetting agents, binders, disintegrants, lubricants, and the like. The fillers may include starch, lactose, mannitol, microcrystalline cellulose, and the like. The absorbents may include calcium sulfate, calcium hydrogen phosphate, calcium carbonate, and the like. The wetting agents may include water, ethanol, and the like. The binders may include hydroxypropyl methylcellulose, polyvinylpyrrolidone (povidon, INN), microcrystalline cellulose, and the like. The disintegrants may include croscarmellose sodium, crospovidone, surfactants, low-substituted hydroxypropyl cellulose, and the like. The lubricants may include magnesium stearate, talcum powder, polyethylene glycol, sodium dodecyl sulfate, colloidal silicon dioxide, talcum powder, and the like. Also, liquids (e.g. aqueous or oily solutions), emulsions, suspensions, or syrups are suitable for oral administration.

For intraperitoneal administration, the S1PR modulators may be formulated, for example, as an injectable solution, emulsion, or suspension. A suitable solvent is, e.g., 3% dimethyl sulfoxide (DMSO).

The pharmaceutical compositions or formulations comprising S1PR modulators may also contain additives used in galenics such as preservation agents, stabilizers, e.g. UV stabilizers, emulsifiers, sweeteners, flavours, coloring agents, salts to change the osmotic pressure, buffers, antioxidants, and the like.

According to the invention, an S1PR modulator (such as fingolimod) for treating Alzheimer's disease may be administered in an amount of about 0.03 mg to about 5 mg S1PR modulator per kg body weight per 1 to 2 days. Examples for suitable amounts within this range are 0.04, 0.05, 0.1, 0.2, 0.3, 0.4, 0.5, 0.6, 0.7, 0.8, 0.9, 1, 1.5, 2, 2.5, 3, 3.5, and 4.5 mg S1PR modulator per kg body weight per 1 to 2 days.

Fingolimod (FTY720) may be administered in an amount of about 0.2 mg to about 1 mg fingolimod per kg body weight per 1 to 2 days. Examples for suitable amounts within this range are 0.3, 0.4, 0.5, 0.6, 0.7, 0.8, and 0.9 mg fingolimod per kg body weight per 1 to 2 days.

The skilled person appreciates that depending on the individual patient situation, other dosages and/or administration periods may be suitable or required.

According to the invention, the administration of an S1PR modulator may take place, for example, over a period of about 1 month to 24 months, preferably over a period of about 1 month to 12 months, e.g., about 1 month to 6 months or about 1 month to 2 months. However, the skilled person appreciates that depending on the individual patient situation, other administration periods may be suitable or required. For example, the application could also be recurring rhythmic such as (daily, or every second day or once a week) application for 2 months, then suspend for 6 months, then again application for 2 months (in a different rhythm, e.g. once a week, every second day, daily, or with a different FTY720 concentration)), or any other related scheme consisting of recurring treatment periods with interleaved pausing of treatment..

In one embodiment of the invention, the patient to be administered with an S1PR modulator may be a patient who has received/is receiving behavioral therapy, physical exercise training, reality orientation training, physiotherapy, occupational therapy, or cognitive training (memory training), or a combination thereof.

In another embodiment of the invention, an S1PR modulator may be used in combination with a drug selected from the group consisting of aducanumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

Aducanumab is an amyloid beta-directed monoclonal antibody approved in the USA in June 2021 under the name Aduhelm (Biogen) through an accelerated procedure for the treatment of Alzheimer's disease.

Riluzole is approved in the USA for the treatment of amyotrophic lateral sclerosis (ALS) and has the formula:

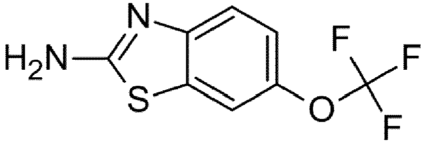

Donepezil is approved in the USA in 2006 for the treatment of mild, moderate, and severe dementia in Alzheimer's disease and has the formula:

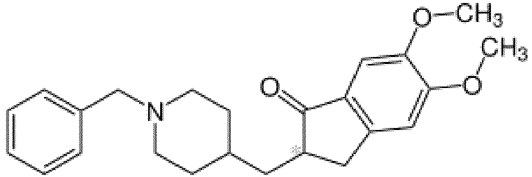

Fluoxetine, sold under the brand names Prozac and Sarafem among others, is an antidepressant. It is on the World Health Organization's List of Essential Medicines and has the formula:

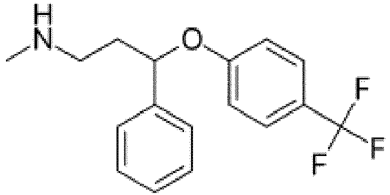

Citalopram, sold under the brand name Celexa among others, is an antidepressant. It is on the World Health Organization's List of Essential Medicines and has the formula:

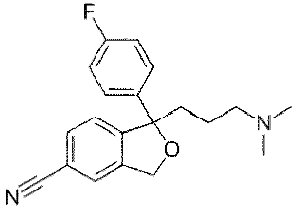

Methylene blue (methylthionium chloride) is a salt of a thiazine dye. It is a tau protein aggregation inhibitor, stabilizes the physiologically normal function of mitochondria and avoids the formation of toxic oxygen radicals. Its formula is:

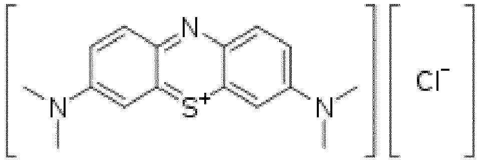

Curcumin is a bright yellow chemical produced by plants of the *Curcuma longa* species. It is a tau protein aggregation inhibitor and has the formula (keto tautomer):

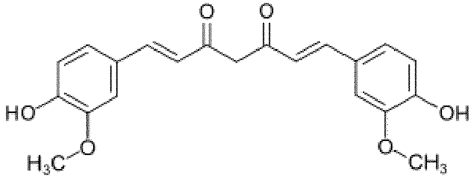

Lithium chloride is a tau protein aggregation inhibitor.

Roscovitine (seliciclib, CYC202) is *inter alia* under investigation for the treatment of chronic inflammation disorders. It is a tau protein aggregation inhibitor and has the formula:

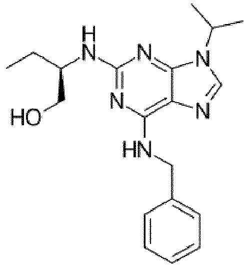

Memantine was approved for medical use in the USA in 2003 and is used to slow the progression of moderate to severe Alzheimer's disease. It is a tau protein aggregation inhibitor and has the formula:

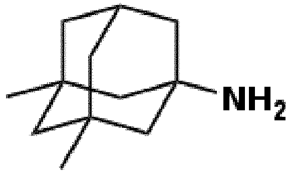

Sodium selenate is a tau protein aggregation inhibitor and has the formula Na₂SeO₄.

Pyridoxamine is one form of vitamin B6. It stabilizes the physiologically normal function of mitochondria and avoids the formation of toxic oxygen radicals. Its formula is:

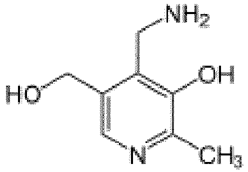

5-aminoimidazole-4-carboxamide-1-β-D-ribofuranoside (Acadesine, AICAR) is used for the treatment of acute lymphoblastic leukemia and has the formula:

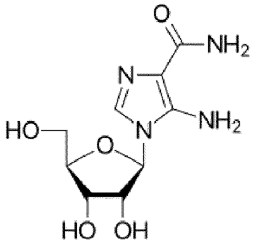

The skilled person appreciates that certain of the above compounds may have different tautomeric forms and/or different optical isomers as a result of one or more asymmetric or chiral carbon atoms. Further, some compounds may display polymorphism. It is also clear that pharmaceutically acceptable salts, esters, solvates, or hydrates of the above compounds as well as prodrugs that may convert *in vivo* into the above compounds may be used in the invention.

Examples for pharmacologically acceptable salts of the above compounds are salts of sufficiently basic compounds of the above formulas with physiologically acceptable mineral acids like hydrochloric, sulfuric or phosphoric acid; or salts of organic acids like acetic, lactic, citric, succinic, fumaric, maleic or salicylic acid. Further, sufficiently acid compounds of the above formulas may form alkali or earth alkaline metal salts, for example, sodium, potassium, lithium, calcium or magnesium salts; ammonium salts; or salts of an organic base, for example, methylamine, dimethylamine, trimethylamine, triethylamine, ethylenediamine, ethanolamine, choline hydroxide, piperidine, morpholine, tris-(2-hydroxyethyl)amine, lysine or arginine salts.

Pharmacologically acceptable esters of the above compounds are esters of the above compounds that can be hydrolyzed by esterase enzymes in the human body into compounds with the above formulas.

Pharmacologically acceptable solvates of the above compounds are complex compounds (adducts) formed by the interaction of the molecules of a solvent with molecules of the above compounds. Hydrates are solvates where the solvent is water.

The first aspect of the present invention can alternatively be formulated as follows: Use of a sphingosine-1-phosphate receptor (S1PR) modulator for the manufacture of a medicament for the treatment of a patient suffering from Alzheimer's dementia. Moreover, the first aspect of the present invention can alternatively be formulated as follows: A method for treating Alzheimer's dementia comprising the steps of
(i) providing an S1PR modulator, and
(ii) administering a patient suffering from Alzheimer's dementia the S1PR modulator in a therapeutically effective amount.

In a second aspect, the invention relates to a combination for use in treating a patient suffering from Alzheimer's dementia, wherein the combination comprises
(i) an S1PR modulator, and
(ii) another therapeutic substance.

According to this aspect of the invention, the S1PR modulator may be selected from the group consisting of fingolimod (FTY720), siponimod (BAF312), ozanimod (RPC1063), ceralifimod (ONO-4641), GSK2018682, ponesimod (ACT128800), and amiselimod (MT-1303). Fingolimod (FTY720) is a preferred S1PR modulator.

As to other preferred embodiments of the S1PR modulator, it is referred to the first aspect of the present invention.

The other therapeutic substance may be selected from the group consisting of aducanumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

The other therapeutic substance is also suitable to treat Alzheimer's dementia in a patient.

Preferred combinations of the S1PR modulator and the other therapeutic substance are:
- Fingolimod and Fluoxetine
- Ozanimod and Fluoxetine
- Siponimod and Fluoxetine
- Fingolimod and Citalopram
- Ozanimod and Citalopram
- Siponimod and Citalopram
- Fingolimod and AICAR
- Ozanimod and AICAR
- Siponimod and AICAR
- Fingolimod and sodium selenite
- Ozanimod and sodium selenite
- Siponimod and sodium selenite
- Fingolimod and AICAR
- Ozanimod and AICAR
- Siponimod and AICAR
- Fingolimod and LiCl
- Ozanimod and LiCl
- Siponimod and LiCl
- Fingolimod and Curcumin
- Ozanimod and Curcumin
- Siponimod and Curcumin

The components of the combination, i.e. the S1PR modulator and the other therapeutic substance, may be administered together or independent from each other (e.g. one after the other).

The second aspect of the present invention can alternatively be formulated as follows: Use of a combination of
(i) an S1PR modulator, and
(ii) another therapeutic substance.
for the manufacture of a medicament for the treatment of a patient suffering from Alzheimer's dementia. Moreover, the second aspect of the present invention can alternatively be formulated as follows: A method for treating Alzheimer's dementia comprising the steps of
(i) providing an S1PR modulator or a combination comprising an S1PR modulator and another therapeutic substance, and
(ii) administering a patient suffering from Alzheimer's dementia the S1PR modulator or the combination comprising an S1PR modulator and another therapeutic substance in a therapeutically effective amount.

In a third aspect, the present invention relates to a pharmaceutical composition comprising the sphingosine-1-phosphate receptor (S1PR) modulator as defined in the first aspect or the combination as defined in the second aspect for use in treating a patient suffering from Alzheimer's dementia.

In a fourth aspect, the present invention relates to method for treating Alzheimer's dementia comprising the steps of
(i) providing an S1PR modulator or a combination comprising an S1PR modulator and another therapeutic substance, and
(ii) administering a patient suffering from Alzheimer's dementia the S1PR modulator or the combination comprising an S1PR modulator and another therapeutic substance in a therapeutically effective amount.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following Figures are merely illustrative of the present invention and should not be construed to limit the scope of the invention as indicated by the appended claims in any way.
FIGURE 1 shows microscope photographs of fingolimod (FTY720) restored spine deficits in CA1 pyramidal neurons in male 15 months old AD model mice.
FIGURE 2 shows the quantitative results of a corresponding dendritic spine density analysis.

### EXAMPLE

The examples given below are for illustrative purposes only and do not limit the invention described above in any way.

The therapeutic effect of fingolimod (FTY720) as an example for an S1PR modulator for use in the treatment of Alzheimer's disease was tested in an established double transgenic amyloid precursor protein/presenilin-1(APP/PS1) Alzheimer's disease (AD) mouse model that starts to develop amyloid-β (Aβ) pathology in the neocortex and hippocampus at two and four months of age, respectively. This AD mouse model is particularly close to human AD pathology. Impaired LTP (long-term potentiation) at Schaffer collateral CA1 synapses in acute slices *ex vivo* is observed at five months in these mice and becomes detectable with *in vivo* recordings beyond six months of age. Moreover, hippocampus-dependent learning deficits in this APP/PS1 mouse model become apparent five to eight months after birth.

To test for a possible amelioration of the AD phenotype at a late symptomatic stage in the above APP/PS 1 mouse model, fingolimod (FTY720) treatment was started at fifteen months of age, when massive loss of synapses and memory has already occurred.

Male APP/PS 1 mice and littermate controls (wild-type healthy mice) were treated with intraperitoneal (i.p.) injections of fingolimod (FTY720; dissolved in 3% DMSO, 1.0 mg/kg body weight) every second day for 1 to 2 months.

The analyses of the animals comprises dendritic spine density analysis, LTP recordings, Morris water maze spatial learning and memory testing, immunohistological stainings, biochemical brain-derived neurotrophic factor (BDNF) quantification, and statistical analysis.

Spine density was determined with Golgi-Cox staining of hippocampal cornu ammonis-1 (CA1) pyramidal neurons. In the APP/PS1 mice, amyloid-β (Aβ) plaques were visualized with the blue fluorescent dye Methoxy X-04.

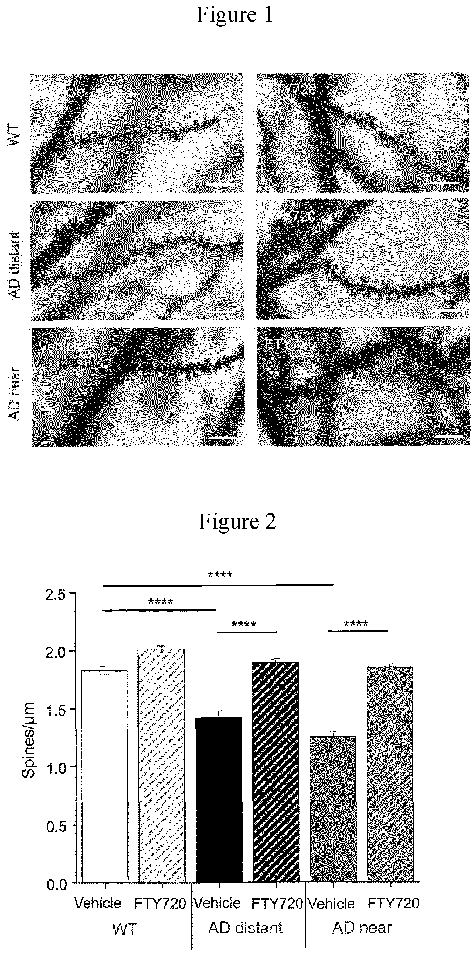

**FIGURE** 1 shows secondary apical dendrites of CA1 pyramidal neurons from 15 months old mice in vehicle and fingolimod treated wild type (WT) animals (WT, upper panel), in APP/PS 1 animals >50 µm away from the closest plaque (AD distant, middle panel), or in APP/PS 1 mice <50 µm away from the nearest plaque (AD near, lower panel). Scale bar: 5 µm in all images.

**FIGURE** 2 shows quantification of spine densities in secondary apical CA1 dendrites shown for the six different groups in Figure 1. Each bar represents the mean value of 10 dendrites from 10 different CA1 pyramidal neurons per animal (three animals per group). Horizontal bars indicate statistical significance between selected groups. Significance of differences was tested with two-way ANOVA followed by Tukey's post hoc test (n = 30 dendrites from three animals per group). Significance level was set to 0.05 (p < 0.05). Different levels of significance are indicated by asterisks, with ^{∗ ∗} = p < 0.01, ^{∗ ∗ ∗} = p < 0.001.

An identical effect of spine rescue in APP/PS1 mice was also observed which much lower doses of fingolimod (FTY720), e.g., 0.2 mg/kg body weight, were applied with the same treatment regime.

The Figures show the complete rescue of spine deficits in dendrites distant to plaques in fingolimod treated APP/PS 1 mice, and the significant amelioration of spine deficits in dendrites close to plaques. The number of synaptic spines can be restored to the level of same-aged healthy mice (wild-type littermates) showing that short-term fingolimod (FTY720) treatment leads to reversion of Alzheimer's disease symptoms when massive loss of synapses and memory has already occurred.

While certain representative embodiments and details have been shown to illustrate the present invention, it will be apparent to those skilled in this art that various changes and modifications can be made and that, within the scope of the appended claims, the invention may be practiced otherwise than as specifically described and claimed.

## Claims

1. A sphingosine-1-phosphate receptor (S1PR) modulator for use in treating a patient suffering from Alzheimer's dementia.

2. The S1PR modulator for use according to claim 1, wherein said S1PR modulator is selected from the group consisting of fingolimod (FTY720), siponimod (BAF312), ozanimod (RPC1063), ceralifimod (ONO-4641), GSK2018682, ponesimod (ACT128800), and amiselimod (MT-1303).

3. The S1PR modulator for use according to claim 2, wherein said S1PR modulator is fingolimod (FTY720).

4. The S1PR modulator for use according to any one of claims 1 to 3, wherein said patient suffering from Alzheimer's dementia is about 40 years old or older.

5. The S1PR modulator for use according to any one of claims 1 to 4, wherein the Alzheimer's dementia is sporadic Alzheimer's dementia or familial Alzheimer's dementia.

6. The S1PR modulator for use according to any one of claims 1 to 5, wherein said treatment is effective to reverse Alzheimer's dementia.

7. The S1PR modulator for use according to any one of claims 1 to 6, wherein said S1PR modulator is to be administered intraperitoneally or orally.

8. The S1PR modulator for use according to any one of claims 1 to 7, wherein said S1PR modulator is to be administered in an amount of between 0.03 mg to 5 mg S1PR modulator per kg body weight per 1 to 2 days.

9. The S1PR modulator for use according to claim 8, wherein said S1PR modulator is fingolimod and is to be administered in an amount of between 0.2 mg to 1 mg fingolimod per kg body weight per 2 days.

10. The S1PR modulator for use according to claims 8 or 9, wherein said administration takes place over a period of about 1 month to 24 months, preferably over a period of about 1 month to 12 months, e.g. about 1 month to 6 months, or about 1 month to 2 months.

11. The S1PR modulator for use according to any one of claims 1 to 10, wherein the patient to be administered is a patient who has received/is receiving behavioral therapy, reality orientation training, physiotherapy, occupational therapy, or cognitive training (memory training), or a combination thereof.

12. The S1PR modulator for use according to any one of claims 1 to 11, wherein the S1PR modulator is used in combination with a drug selected from the group consisting of aducanumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

13. A combination for use in treating a patient suffering from Alzheimer's dementia, wherein the combination comprises
(i) an S1PR modulator, and
(ii) another therapeutic substance.

14. The combination for use according to claim 13, wherein said S1PR modulator is selected from the group consisting of fingolimod (FTY720), siponimod (BAF312), ozanimod (RPC1063), ceralifimod (ONO-4641), GSK2018682, ponesimod (ACT128800), and amiselimod (MT-1303).

15. The combination for use according to claim 14, wherein said S1PR modulator is fingolimod (FTY720).

16. The combination for use according to any one of claims 13 to 15, wherein the other therapeutic substance is selected from the group consisting of aducanumab, riluzole, donepezil, fluoxetine, citalopram, methylene blue, curcumin, lithium chloride, roscovitine, memantine, sodium selenate, pyridoxamine (vitamin B6), and 5-aminoimidazole-4-carboxamide-1-β-D-ribofuranoside (AICAR).

17. A pharmaceutical composition comprising the sphingosine-1-phosphate receptor (S1PR) modulator as defined in claims 1 to 12 or the combination as defined in claims 13 to 16 for use in treating a patient suffering from Alzheimer's dementia.

18. A method for treating Alzheimer's dementia comprising the steps of
(i) providing an S1PR modulator or a combination comprising an S1PR modulator and another therapeutic substance, and
(ii) administering a patient suffering from Alzheimer's dementia the S1PR modulator or the combination comprising an S1PR modulator and another therapeutic substance in a therapeutically effective amount.
